# EUROPEAN PATENT APPLICATION

(11) **EP 1 275 403 A1**
(43) Date of publication of application: **15.01.2003**
(21) Application number: 01126692.1
(22) Date of filing: 08.11.2001
(51) Int. Cl.: A61L 15/46, A61L 15/44

(54) **Additive for a sanitary napkin and a method of adding the same**

(30) Priority: 10.07.2001 CN 01106900
(71) Applicant: Shan, Lin I., Taitung City, Taitung Hsien, Taiwan (CN)
(72) Inventor: Shan, Lin I., Taitung City, Taitung Hsien, Taiwan (CN)
(74) Representative: Haft, von Puttkamer, Berngruber, Czybulka

(57) **Abstract**

This invention relates to an additive for a sanitary napkin and a method of adding the same, and is directed to a liquid mixture containing Borneo camphor, menthol, alum, sodium salicylate, camphor and aloe vera at a specific proportion. In use, a face layer disposed between absorbent layer and surface layer of the sanitary napkin is immersed in the mixture such that the mixture can completely and evenly penetrate into the face layer. The mixture is further sprayed onto the surface layer of the sanitary napkin. After drying, the face and surface layers are coupled with a bottom layer and a high polymer body to thereby obtain a sanitary napkin that can promote blood circulation, enhance absorption power of the sanitary napkin, prevent breeding of germs, and make the user feel dry and comfortable.

## Description

### BACKGROUND OF THE INVENTION

### (a) Field of the Invention:

The invention relates to an additive for a sanitary napkin and a method of adding the same, in which a mixture containing Borneo camphor, menthol, alum, sodium salicylate, camphor and aloe vera at a specific proportion is allow to penetrate into a sanitary napkin via immersion, spraying, spreading, and cold-drying so as to evenly distribute over the sanitary napkin. In use, due to effects of the medicines, blood circulation of the user and the absorption power of the sanitary napkin can be enhanced, breeding of germs can be prevented, and the user feels dry and comfortable.

### (b) Description of the Prior Art:

Sanitary napkins are used by female users during the menstrual cycle, and sanitary pads are used outside the menstrual cycle. Both of them are designed to prevent leakage and have a surface layer which is in contact with the user's skin and which should make the user feel dry. However, conventional sanitary napkins and pads do not have other functions than absorbing the menstrual discharge. For female users, their reproductive organs are more susceptible to germs during the menstrual cycle, and there may be undesirable odor as a result of prolonged contact with the sanitary napkin or pad. Moreover, conventional sanitary napkins and pads are not capable of killing germs or removing bad odor.

### SUMMARY OF THE INVENTION

The object of this invention is to provide an additive for a sanitary napkin, which is a liquid mixture containing 10-15% weight % of Borneo camphor, 15-20% weight % of menthol, 10-20% weight % of alum, 10-15% weight % of sodium salicylate, 10-15% wt % of camphor, and 10-15% wt % of aloe vera. In use, due to the effects of the medicines, the blood circulation and the absorption power of the sanitary napkin can be enhance, breeding of germs can be prevented, and the user can feel dry and comfortable.

Another object of the invention is to provide a method of adding the additive to the sanitary napkin, in which a face layer disposed between a surface layer and an absorbent layer is immersed in a liquid mixture containing Borneo camphor, menthol, alum, sodium salicylate, camphor and aloe vera. as well as solvent. The mixture is then sprayed onto the surface layer. After drying of the face layer and surface layer, the face layer and surface layer is coupled with a bottom layer and a high polymer body to obtain a sanitary napkin that can promote blood circulation, enhance absorption power of the sanitary napkin, prevents breeding of germs, and make the user feel dry and comfortable.

### BRIEF DESCRIPTION OF THE DRAWINGS

The foregoing and other features and advantages of the present invention will be more clearly understood from the following detailed description and the accompanying drawings, in which,

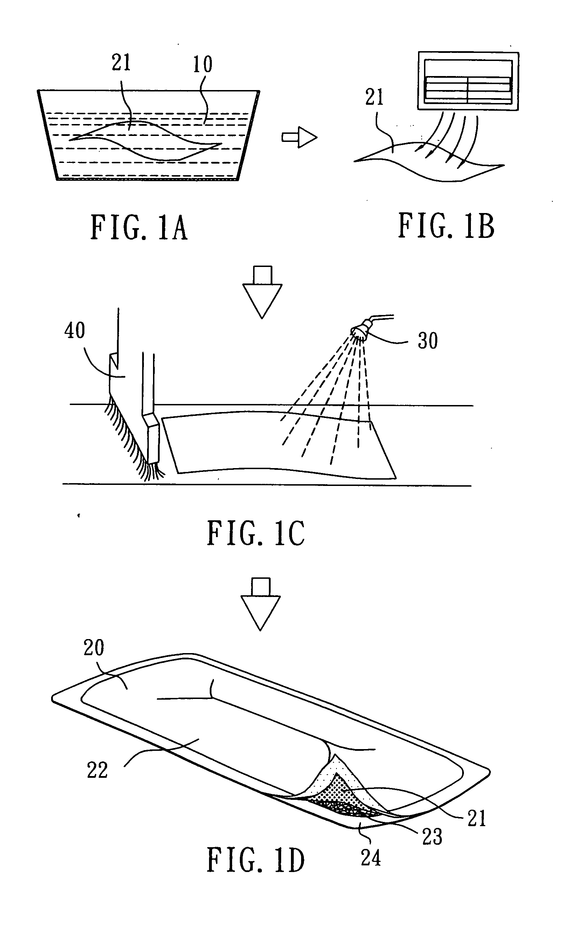

Figure 1A is a schematic view illustrating the first step of the adding method according to the invention;
Figure 1B is a schematic view illustrating the second step of the adding method according to the invention;
Figure 1C is a schematic view illustrating the third step of the adding method according to the invention; and
Figure 1D illustrates a product obtained via the adding method according to the invention.

### DETAILED DESCRIPTION OF THE PREFERRED EMBODIMENT

The invention relates to an additive for sanitary napkins, which is directed to a liquid mixture 10 composed of 10-15% weight % of Borneo camphor, 15-20% weight % of menthol, 10-20% weight % of alum, 10-15% weight % of sodium salicylate, 10-15% wt % of camphor, and 10-15% wt % of aloe vera. Of the mixture 10 (as shown in Figure 10A), according to the Outline of Medicinal Herbs, Borneo camphor mainly treats labor pain, can remove abdominal gas, wetness and heat, and is used as pain killer and vermicide; menthol is bitter and cool in nature, and can remove wetness and heat, the stems and leaves thereof are moderate and non-toxic, can cure liver gas and typhoid, perspiring, and can cool, kill pain, stop itchiness, and alleviate discomfort; alum can alleviate toxicity, kills worms and germs, soothes and stops itchiness; sodium salicylate can alleviate inflammation and soothes pain; camphor is snowy white and mainly treats body gas, and can stop itchiness and pain; aloe vera alleviates inflammation and has moisturizing effect. For better understanding the effects of the respective component, a table is set forth below:

| Component | Effect |
|---|---|
| Borneo camphor | Removes heat and kills pain, vermicide |
| Menthol | Cools, kills pain, stops itchiness, alleviates discomfort |
| Alum | Alleviates toxicity, kills worms and germs, soothes, stops |
| | Itchiness |
| Sodium salicylate | Alleviates inflammation and soothes pain |
| Camphor | Stops itchiness and kills pain |
| Aloe vera | moisturizes and alleviates inflammation |

The method of adding the additive of this invention is as follows:

Referring to Figures 1A, 1B, 1C, 1D, a face layer between surface layer 22 and absorbent layer 23 of a sanitary napkin 20 is immersed in the liquid mixture 10 such that the mixture 10 can the mixture 10 is sprayed using a spray head 30 onto a surface layer 22. The mixture 10 on the surface layer 22 is spread even using a brush 40. The surface layer 22 is dried using cold air (see Figure 1C).

Lastly, similar to the conventional method of making sanitary napkins, the face layer 21, surface layer 22, a bottom layer 24, and absorbent layer 23 are stacked in sequence, and is coupled integrally using fusion so as to obtain a sanitary napkin product (see Figure 1D).

In use, since the additive 10 of this invention penetrates into the surface layer 22 and face layer 21 that are in contact with the user's skin, it can penetrate into the user via vent holes 25. As the mixture 10 has the effects of removing heat, killing pain and germs, soothing, stopping itchiness, alleviating discomfort, alleviating toxicity, killing worms, alleviating inflammation and moisturizing, it can enhance the absorptive power of the sanitary napkin 20 to prevent breeding of germs and make the user feel dry and comfortable.

In sum, the major effect of this invention is to enhance the blood circulation of the user in part during or outside the menstrual cycle, and to remove wetness and prevent breeding of germs.

Although the present invention has been illustrated and described with reference to the preferred embodiment thereof, it should be understood that it is in no way limited to the details of such embodiment but is capable of numerous modifications within the scope of the appended claims.

## Claims

1. An additive for a sanitary napkin, which is a liquid mixture containing 10-15% weight % of Borneo camphor, 15-20% weight % of menthol, 10-20% weight % of alum, 10-15% weight % of sodium salicylate, 10-15% wt % of camphor, and 10-15% wt % of aloe vera.

2. A method of adding an additive to a sanitary napkin, comprising the steps of: immersing a face layer disposed between a surface layer and an absorbent layer of the sanitary napkin in a liquid mixture of Claim 1 such that the mixture can completely and evenly penetrate into the face layer, and subsequently drying the face layer using cold air; and spraying the mixture onto the surface layer and spreading the mixture on the surface layer evenly using a brush, and drying the surface layer using cold air.
